# EUROPEAN PATENT APPLICATION

(11) **EP 3 107 148 A1**
(43) Date of publication of application: **21.12.2016**
(21) Application number: 16171932.3
(22) Date of filing: 30.05.2016
(51) Int. Cl.: H01Q 1/27, A61N 1/372, H01Q 1/36, H01Q 11/08

(54) **IMPLANTABLE MEDICAL DEVICE INCLUDING A HIGH-FREQUENCY ELECTRONIC ELEMENT**

(30) Priority: 19.06.2015 US 201562181776 P
(71) Applicant: BIOTRONIK SE & Co. KG, 12359 Berlin (DE)
(72) Inventor: Brown, James, Tigard, Oregon 97224 (US); Austin, Eric, Portland, Oregon 97221 (US)
(74) Representative: Galander, Marcus

(57) **Abstract**

The invention relates to an antenna arrangement comprising a generally helical antenna body (10) arranged about a longitudinal axis (30) and enclosing a generally helical signal lead body (20), the antenna body (10) and the signal lead body (20) having opposing rotational directions.

Further the invention relates to an implantable electronic device (100, 200, 300), comprising a header (110, 210, 310) attached to a housing (120, 220, 320), wherein an antenna arrangement is arranged in the header (110, 210, 310), the antenna arrangement comprising a generally helical antenna body (10) arranged about a longitudinal axis (30) and coaxially enclosing a generally helical signal lead body (20), the antenna body (10) and the signal lead body (20) having opposing rotational directions.

## Description

The invention relates to an implantable medical device including a high frequency electronic element.

Several structures for size reduction of antennas in small portable or implantable devices are generally known in the art.

US 6,708,065 B2 discloses an implantable medical device in which an antenna is embedded in a dielectric compartment. For saving space the antenna may be a helical antenna which is shorter than an elongated dipole or monopole antenna. When the radius of the helical coiling is small enough, the radiation pattern is approximately the same as of an elongated dipole or monopole antenna.

US 6,075,501 A discloses an arrangement of two helical antennas, one on each side of a substrate, which have slightly different inclinations. The antenna arrangement is proposed for portable devices such as mobile phones.

The primary requirement of radio frequency (RF) systems for implanted medical devices is the reception of RF signals from a (sometimes distant) radiator. Absorption of the RF energy by biological tissues often means the received RF signals are very low in amplitude. Therefore, in order to operate with acceptable range, the implanted RF system must be able to detect signals that are very close to the environmental noise floor. Noise can be generated by the device itself and injected into the RF system by the signal lead or leads, thereby raising the noise floor, which is the input signal level, at which the signal, that is intended to be received, cannot be distinguished from other sources of electromagnetic radiation/noise, and decreasing the range.

It is an object of the invention to provide an antenna arrangement which has a reduced sensibility to noise introduced to the system by the signal lead.
Another object of the invention is to provide an implantable electronic device having such an antenna arrangement.

The objects are achieved by the features of the independent claims. The other claims, the drawings and the specification disclose advantageous embodiments of the invention.

An antenna arrangement is proposed comprising a generally helical antenna body arranged about a longitudinal axis and enclosing a generally helical signal lead body, the antenna body and the signal lead body having opposing rotational directions and thereby preventing the injection of noise into the RF system by the signal lead.

The expression "generally helical" means that the cross section of the windings is not necessarily circular but may be elliptical or may take any number of polygonal cross-sections, for example a rectangular cross section, or the like. Further, the generally helical antenna body and the generally helical signal lead body may have helices with different cross sections, for example an helical antenna body with a rectangular cross section is enclosing a helical signal lead body with a circular cross section or vice versa.

Advantageously, the inventive solution allows for constructing the antenna as a space efficient helix antenna, and to construct signal leads such as therapy conductors in a concentric, helical shape of the opposite orientation, for instance, a right-hand wound helix for the antenna body and a left-hand wound helix for the signal lead body or vice versa. Good performance is also derived, if the signal lead is arranged eccentrically within the helical antenna. Concentric or eccentric construction or arrangement means, that the longitudinal axes of the helical antenna as well as of the helical signal lead are situated concentrically or eccentrically to each other in a parallel manner. In either concentric or eccentric configurations, different cross sections of both the helical antenna and the signal lead are possible.

In another configuration the axis of both the helical antenna and the helical signal lead may be not parallel to each other, which means, they are tilted from each other. In this configuration, both the helical antenna and the helical signal lead may also have different cross sections and may also be arranged concentrically or eccentrically.

Depending on the medical application, the signal lead may be a sense lead, an electrode lead like a stimulation lead, or the like.

It is possible to electromagnetically decouple the antenna from the other conductive material enclosed by the antenna body that may introduce electromagnetic interference (EMI). Thus, by utilizing this invention the number of errors during RF data transmission may be reduced.

In order to obtain desirable RF system performance, the antenna is designed to be as large as possible. This can make the antenna body larger than it is desirable. Because of the well-known fundamental limit of antenna gain with physical size, optimizing the antenna for the highest gain possible is not feasible as devices become miniaturized. By designing a smaller antenna body volume, it follows that the maximum antenna size possible is also smaller, causing the maximum possible signal strength received by this antenna to be lower. Favorably, a solution whereby the sensitivity to noise is reduced is a desirable solution for optimizing the performance of the RF systems utilizing small antennas.

According to an advantageous embodiment, the antenna body and the signal lead body may be arranged coaxially, thus providing further reduction of errors during RF data transmission.

In another embodiment, the antenna body and the signal lead body may be arranged eccentrically.

According to an advantageous embodiment, the antenna body may have a polygonal cross-section, preferably a generally rectangular cross section. The cross section may have straight portions at the circumference and rounded edges.

According to an advantageous embodiment, the signal lead body may have a generally round or polygonal cross-section. Preferably the signal lead body may have an oval cross section. Thus, the antenna body cross section circumscribes a comparatively large volume.

According to an advantageous embodiment, the antenna body and/or the signal lead body may be each disposed on separate carriers. For example one carrier can be provided for the antenna body and another carrier for the signal lead body. Both carriers may have at least one surface, on which each the antenna body and the signal lead body is helically wound around. In particular, on the at least one surface an attachment means may be situated, which helps to form and maintain a defined helical shape of each of the antenna body and the signal lead body. One example for an attachment means is a groove, for example a notched groove, which is helically formed on the at least one surface, wherein each the antenna body and the signal lead body is situated and attached. The pitch and relative position of antenna leads and signal leads can be manufactured in a reproducible manner. The groove is for example milled or casted with a suitable mold. Alternatively the antenna body and the signal lead body are disposed on opposing sides of one carrier (a single carrier). For example, the antenna body and the signal lead body are helically wound around on the opposing sides (surfaces) of the carrier. In particular, on each of the opposing surfaces of the carrier an attachment means may be situated, which helps to form and maintain a defined helical shape of each of the antenna body and the signal lead body. One example for an attachment means is a groove (notched groove), which is helically formed on each of the opposing surfaces of the carrier, wherein each the antenna body and the signal lead body is situated and attached. In particular, the carrier may be a flexible substrate. The antenna and signal leads may be deposited on the substrate by conventional thin film deposition techniques such as vacuum deposition, screen printing and the like. In case the carrier is a flexible circuit like a printed circuit board (PCB), the antenna and signal lead material may be plated or etched on the surface of the PCB.

According to another aspect of the invention an implantable electronic device is proposed, comprising a header attached to a housing, wherein an antenna arrangement is arranged in the header, the antenna arrangement comprising a generally helical antenna body arranged about a longitudinal axis and coaxially enclosing a generally helical signal lead body, the antenna body and the signal lead body having opposing rotational directions.

It is possible to electromagnetically decouple the antenna from the signal or signal leads within the header that may introduce electromagnetic interference (EMI). Favorably, by utilizing this invention the number of errors during RF data transmission may be reduced, which will reduce the number of "re-try" events, which will save battery power and increase implant longevity. An additional advantage of the reduced battery power required by the RF system is that a smaller sized battery can be utilized, thereby reducing the implant size. The device will be easier to implant, with increased patient comfort and lower cost. Reduced size of the device header reduces the total implant size.

According to an advantageous embodiment, the antenna body and the signal lead body may have virtually the same pitch size. This is favorable for reducing the electromagnetic effect of the signal lead body on the antenna.

According to an advantageous embodiment, the signal lead body may be connected to an electrode structure at an outer surface of the header. A secure electrical connection is possible.

According to an advantageous embodiment, the signal lead body may have an elongation extending beyond a region surrounded by the antenna body. In particular, the elongation may be arranged between the region surrounded by the antenna body and the electrode structure. The signal lead may serve as sense lead which extends beyond the helical portion in order to attach to the electrode structure. The elongated sense lead allows for a longer sense vector when attached to sense electrode.

According to an advantageous embodiment, the antenna body and/or the signal lead body may be each disposed on separate carriers. Alternatively the antenna body and the signal lead body may be disposed on opposing sides of one carrier (a single carrier). The pitch and relative position of antenna leads and signal leads can be manufactured in a reproducible manner. When the antenna body and the signal lead body are disposed on opposing sides of one carrier, the carrier may be a flexible substrate. The antenna and signal leads may be deposited on the substrate by conventional thin film deposition techniques such as vacuum deposition, screen printing and the like. Also, in case the carrier is a PCB, the antenna and signal lead material may be plated or etched on the surface of the PCB.

According to an advantageous embodiment, the antenna body may have a generally rectangular cross section. Favorably, the signal lead may have a generally oval cross section.

The present invention together with the above-mentioned and other objects and advantages may best be understood from the following detailed description of the embodiments, but not restricted to the embodiments, wherein is shown in:
- Fig. 1: a header part of a medical electronic device comprising an antenna arrangement according to an embodiment of the invention;
- Fig. 2: an isometric view of the antenna arrangement of Figure 1;
- Fig. 3: a cross sectional view of the antenna arrangement of Figure 1;
- Fig. 4: a header part of a medical electronic device comprising an antenna arrangement according to an embodiment of the invention;
- Fig. 5: a carrier body for the antenna arrangement of Figure 4;
- Fig. 6: carrier bodies for the antenna arrangement of Figure 4;
- Fig. 7: a flexible carrier with antenna leads and signal leads deposited on; and
- Fig. 8: a header part of a medical electronic device comprising the antenna arrangement of Figure 7 according to an embodiment of the invention.

In the drawings, like elements are referred to with equal reference numerals. The drawings are merely schematic representations, not intended to portray specific parameters of the invention. Moreover, the drawings are intended to depict only typical embodiments of the invention and therefore should not be considered as limiting the scope of the invention.

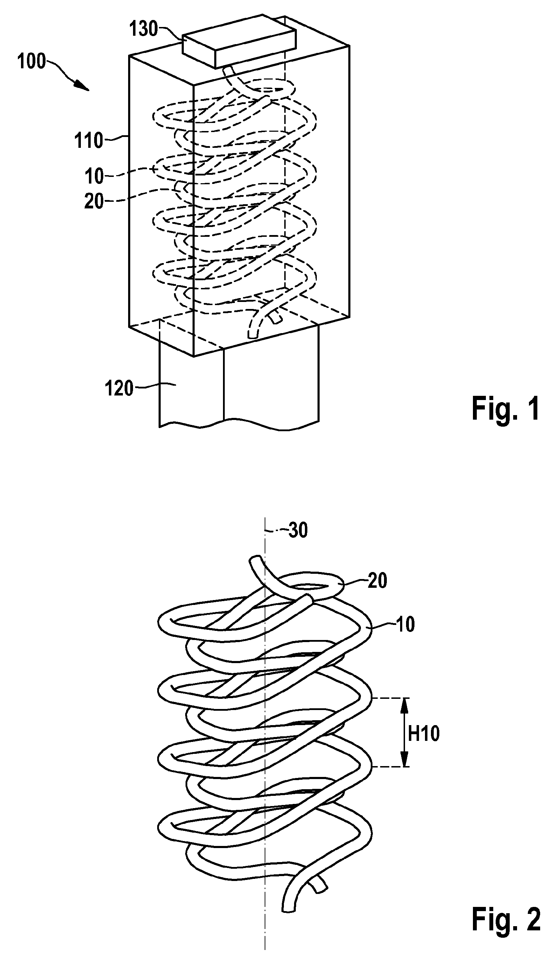

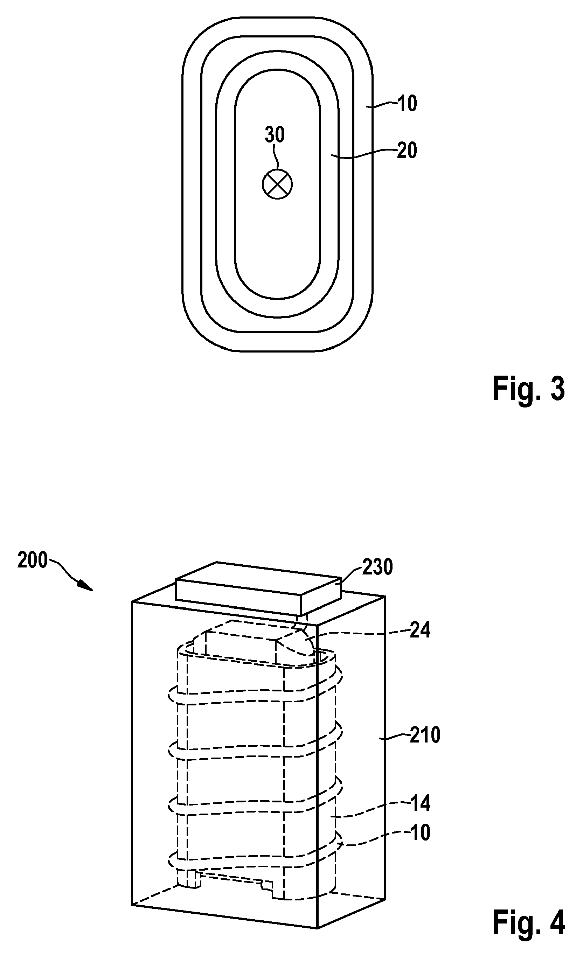

Figures 1, 2 and 3 depict a first embodiment of the invention. Figure1 shows a header part 110 of a medical electronic device 100 comprising an antenna arrangement according to an embodiment of the invention. Figure 2 shows the antenna arrangement in detail and Figure 3 shows cross sections of the antenna body 10 and the signal lead body 20. The windings of the antenna body 10 and the signal lead body 20 have opposite directions. The long side portions of the windings of antenna and signal leads are arranged in parallel, while the narrow, bended side portions are arranged so that an axial position of a bend of the signal lead body 20 is located in a space between two adjacent bends of the antenna body 10. The antenna body 10 and the signal lead body 20 have the same pitch size H10.

The header part 110 is shown in a semi-transparent manner to show the antenna arrangement inside the header part. The header part 110 is attached to a housing 120 the top of which is illustrated and which includes usual and necessary electronics and processor units and the like as well known in the art. A battery (not shown) may be attached to the housing 120 or integrated in the housing 120.

The antenna arrangement comprises a generally helical antenna body 10 arranged about a longitudinal axis 30 and enclosing a generally helical signal lead body 20 in a coaxial manner. The antenna body 10 and the signal lead body 20 having opposing rotational directions.

The antenna arrangement extends between the housing and an electrode structure 130 arranged at the top of the header part 110. The helical arrangement allows for miniaturization of the antenna body 10 and thus a miniaturization of the header part 110.

In addition the signal lead body 20 may have an elongation extending beyond a helical region surrounded by the antenna body 10. In this case the header part 110 has an elongated top part carrying an electrode structure, for example at its top.

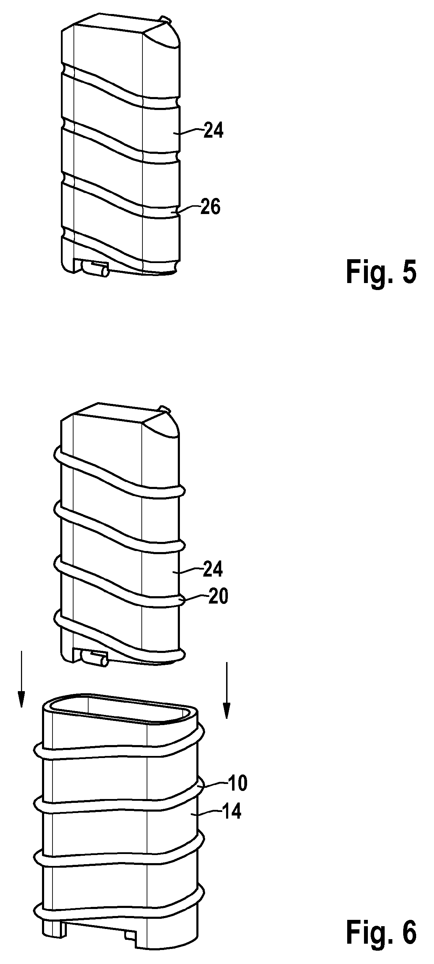

Figures 4, 5 and 6 depict a further embodiment of the invention. Figure 4 shows a header part 210 (shown in a semi-transparent manner) of a medical electronic device 200 comprising an antenna arrangement according to an embodiment of the invention. The header part 200 is attached to a housing (not shown) and has an electrode structure 230 at its top. Figure 5 shows a carrier body 24 for the antenna arrangement of Figure 4, and Figure 6 shows carrier bodies 14, 24 for the antenna arrangement of Figure 4.

The windings of the antenna body 10 and the signal lead body 20 have opposite directions. The long side portions of the windings of antenna and signal leads are arranged in parallel, while the narrow, bended side portions are arranged so that an axial position of a bend of the signal lead body 20 is located in a space between two adjacent bends of the antenna body 10. The antenna body 10 and the signal lead body 20 have the same pitch size.

The carrier 24 of the signal lead body 20 has a helical groove 26 in which the lead wire is disposed. The antenna body 10 is disposed on the antenna body carrier 14. The antenna body carrier 14 can accommodate the carrier 24 of the signal lead body 20 so that the antenna and the signal lead are arranged in a coaxial manner.

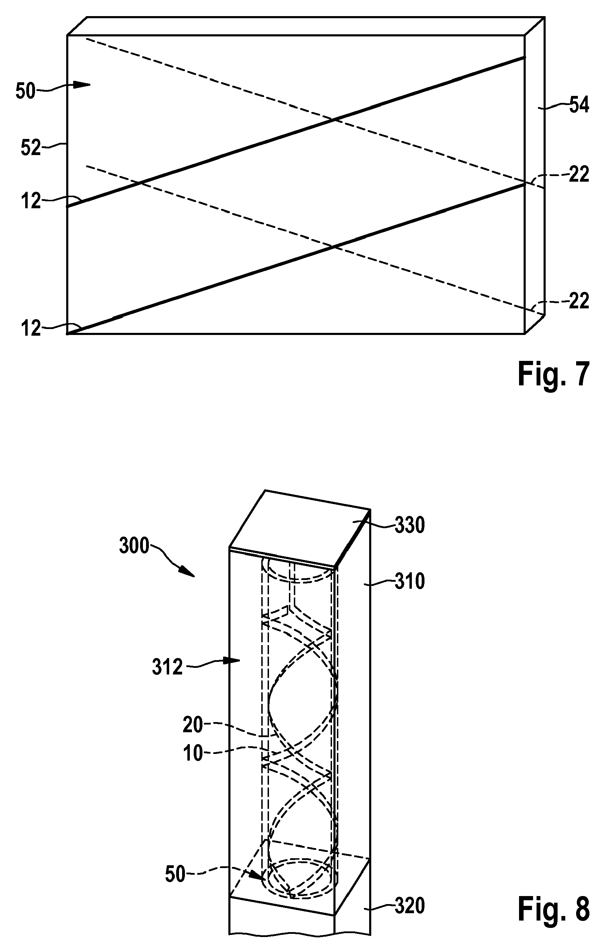

Figures 7 and 8 illustrate a further embodiment of the invention. Figure 7 shows a flexible carrier 50 with antenna lead portions 12 and signal lead portions 22 deposited on opposing surfaces of the carrier 50. Figure 8 shows a header part 310 of a medical electronic device 300 comprising the antenna arrangement of Figure 7 according to an embodiment of the invention. The windings of the antenna body 10 and the signal lead body 20 have opposite directions. The long side portions of the windings of antenna and signal leads are arranged in parallel, while the narrow, bended side portions are arranged so that an axial position of a bend of the signal lead body 20 is located in a space between two adjacent bends of the antenna body 10. The antenna body 10 and the signal lead body 20 have the same pitch size.

The antenna body 10 and the signal lead body 20 are formed by connecting the two opposing edges 52 and 54 and connecting the separate antenna lead portions 12 with each other as well as the signal lead portions 22 forming electric connections between the portions 12 as well as between the portions 22, e.g. by soldering or welding the single portions. Preferably the method of laser or resistance welding is used to form electrical connections between the portions 12 and 22. Further preferred, if the flexible carrier 50 is a PCB, the electric connections between the portions 12 and 22 may be made during PCB manufacturing, for example using layer-to-layer plated vias.

## Claims

1. An antenna arrangement comprising a generally helical antenna body (10) arranged about a longitudinal axis (30) and enclosing a generally helical signal lead body (20), the antenna body (10) and the signal lead body (20) having opposing rotational directions.

2. The antenna arrangement according to claim 1, wherein the antenna body (10) and the signal lead body (20) are arranged coaxially.

3. The antenna arrangement according to claim 1 or 2, wherein the antenna body (10) has a polygonal cross-section, preferably a generally rectangular cross section.

4. The arrangement according to any one of the preceding claims, wherein the signal lead body (20) has a generally round cross section or polygonal cross-section, preferably a generally oval cross-section.

5. The arrangement according to any one of the preceding claims, wherein the antenna body (10) and/or the signal lead body (20) are each disposed on separate carriers (14, 24).

6. The arrangement according to any one of the claims 1 to 4, wherein the antenna body (10) and the signal lead body (20) are disposed on opposing sides of a carrier (50).

7. The arrangement according to claim 6, wherein the carrier (50) is a flexible substrate.

8. An implantable electronic device (100, 200, 300), comprising a header (110, 210, 310) attached to a housing (120, 220, 320), wherein an antenna arrangement is arranged in the header (110, 210, 310), the antenna arrangement comprising a generally helical antenna body (10) arranged about a longitudinal axis (30) and coaxially enclosing a generally helical signal lead body (20), the antenna body (10) and the signal lead body (20) having opposing rotational directions.

9. The device according to claim 8, wherein the antenna body (10) and the signal lead body (20) have virtually the same pitch size.

10. The device according to any one of the claims 8 to 9, wherein the signal lead body (20) is connected to an electrode structure (130, 230, 330) at an outer surface of the header (110, 210, 310).

11. The device according to any one of the claims 8 to 10, wherein the signal lead body (20) has an elongation (26) extending beyond a region surrounded by the antenna body (10).

12. The device according to claim 11, wherein the elongation (26) is arranged between the region surrounded by the antenna body (10) and the electrode structure (230).

13. The device according to any one of the claims 8 to 12, wherein the antenna body (10) and/or the signal lead body (20) are each disposed on separate carriers (14, 24) or wherein the antenna body (10) and the signal lead body (20) are disposed on opposing sides of a carrier (50).

14. The device according to any one of the claims 8 to 13, wherein the antenna body (10) has a generally rectangular cross section.
